(19) **Europäisches Patentamt**
**European Patent Office**
**Office européen des brevets**

(11) **EP 3 099 240 B1**

(12) **FASCICULE DE BREVET EUROPEEN**

(45) Date de publication et mention de la délivrance du brevet:
**28.02.2024 Bulletin 2024/09**

(21) Numéro de dépôt: **15701563.7**

(22) Date de dépôt: **12.01.2015**

(51) Classification Internationale des Brevets (IPC):
**A61B 8/00** (2006.01)      **A61B 8/08** (2006.01)
**G01N 29/07** (2006.01)      **G16H 50/30** (2018.01)

(52) Classification Coopérative des Brevets (CPC):
**A61B 8/485; A61B 8/085; A61B 8/0858;**
**A61B 8/0883; A61B 8/5223; G16H 50/30;**
A61B 8/4281

(86) Numéro de dépôt international:
**PCT/FR2015/050058**

(87) Numéro de publication internationale:
**WO 2015/114232 (06.08.2015 Gazette 2015/31)**

(54) **PROCEDE ET DISPOSITIF ULTRASONORE DE CARACTERISATION DES MILIEUX MOUS ANISOTROPES, ET ENSEMBLE DE SONDE ULTRASONORE POUR UN TEL DISPOSITIF DE CARACTERISATION**

ULTRASCHALLVERFAHREN UND VORRICHTUNG ZUR CHARAKTERISIERUNG SCHWACHER ANISOTROPER MEDIEN UND ULTRASCHALLSONDENANORDNUNG FÜR SOLCH EINE CHARAKTERISIERUNGSVORRICHTUNG

ULTRASONIC METHOD AND DEVICE FOR CHARACTERISING WEAK ANISOTROPIC MEDIA, AND ULTRASONIC PROBE ASSEMBLY FOR SUCH A CHARACTERISATION DEVICE

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **31.01.2014 FR 1400265**

(43) Date de publication de la demande:
**07.12.2016 Bulletin 2016/49**

(72) Inventeurs:
• **TANTER, Mickaël**
  **F-92220 Bagneux (FR)**
• **PERNOT, Mathieu**
  **F-75004 Paris (FR)**
• **FINK, Mathias**
  **F-92190 Meudon (FR)**
• **GENNISSON, Jean-Luc**
  **F-95000 Cergy (FR)**

(74) Mandataire: **Lavoix**
  **2, place d'Estienne d'Orves**
  **75441 Paris Cedex 09 (FR)**

(73) Titulaires:
• **Centre National de la Recherche Scientifique (C.N.R.S.)**
  **75016 Paris (FR)**
• **INSERM - Institut National de la Santé et de la Recherche Médicale**
  **75013 Paris (FR)**
• **Ecole Supérieure de Physique et de Chimie Industrielles de la Ville de Paris**
  **75005 Paris (FR)**
• **Université Paris Cité**
  **75006 Paris (FR)**

(56) Documents cités:
**US-A1- 2012 116 220**

• **WEI-NING LEE ET AL: "Mapping Myocardial Fiber Orientation Using Echocardiography-Based Shear Wave Imaging", IEEE TRANSACTIONS ON MEDICAL IMAGING, IEEE SERVICE CENTER, PISCATAWAY, NJ, US, vol. 31, no. 3, 2 mars 2012 (2012-03-02), pages 554-562, XP011491045, ISSN: 0278-0062, DOI: 10.1109/TMI.2011.2172690**

**(Cont. page suivante)**

- **EMILIE MACE ET AL: "In Vivo Mapping of Brain Elasticity in Small Animals Using Shear Wave Imaging", IEEE TRANSACTIONS ON MEDICAL IMAGING, IEEE SERVICE CENTER, PISCATAWAY, NJ, US, vol. 30, no. 3, 2 mars 2011 (2011-03-02), pages 550-558, XP011349049, ISSN: 0278-0062, DOI: 10.1109/TMI.2010.2079940**
- **BRUM J ET AL: "evaluation of the elastic anisotropy of the human Achilles tendon using shear wave dispersion analysis", PHYSICS IN MEDICINE AND BIOLOGY, INSTITUTE OF PHYSICS PUBLISHING, BRISTOL GB, vol. 59, no. 3, 17 janvier 2014 (2014-01-17), pages 505-523, XP020256772, ISSN: 0031-9155, DOI: 10.1088/0031-9155/59/3/505 [extrait le 2014-01-17]**

**Description**

DOMAINE DE L'INVENTION

L'invention concerne les procédés et dispositifs de caractérisation des milieux mous anisotropes, et les ensembles de sondes ultrasonores pour de tels dispositifs de caractérisation.

ARRIERE-PLAN DE L'INVENTION

Il a déjà été proposé de caractériser des milieux mous anisotropes comportant des fibres, tels que des muscles squelettiques ou le myocarde, par imagerie de la propagation d'ondes de cisaillement dans ces milieux. Plus particulièrement, il a été proposé de mesurer la valeur du module de cisaillement de ces milieux le long des fibres et perpendiculairement aux fibres, en faisant propager successivement des ondes de cisaillement dans le milieu et en imageant leur propagation à chaque fois dans une direction différente, grâce à une barrette de transducteurs ultrasonore que l'on fait tourner de quelques degrés entre deux émissions d'onde de cisaillement (W.-N. Lee, M. Pernot, M. Couade, E. Messas, P. Bruneval, A. Bel, A. A. Hagège, M. Fink, and M. Tanter, "Mapping Myocardial Fiber Orientation Using Echocardiography-Based Shear Wave Imaging," IEEE Trans. Med. Imaging, vol. 31, pp. 554-562, 2012). On détermine ainsi la vitesse de propagation des ondes de cisaillement dans chaque direction. Par décomposition des mesures de vitesse en valeurs singulières, on peut obtenir à la fois la direction des fibres et les modules de cisaillement le long des fibres et perpendiculairement aux fibres, comme enseigné par *Lee* et *al.* (W.-N. Lee, B. Larrat, M. Pernot, and M. Tanter, "Ultrasound Elastic Tensor Imaging: Comparison with MR Diffusion Tensor Imaging in the Myocardium," Physics in Medicine and Biology, vol. 57, pp. 5075-5095, 2012).

Ces procédés connus présentent toutefois l'inconvénient d'être relativement lents à mettre en oeuvre. De ce fait, ils ne permettent notamment pas une caractérisation de milieux fibreux mobiles tels que le myocarde d'un patient ou un muscle squelettique d'un patient.

OBJETS ET RESUME DE L'INVENTION

La présente invention a notamment pour but de pallier ces inconvénients.

A cet effet, l'invention propose un procédé de caractérisation d'un milieu mou anisotrope comprenant au moins une partie comportant des fibres et présentant une surface extérieure, ce procédé comprenant les étapes selon la revendication 1.

Grâce à ces dispositions, on peut accéder instantanément à la direction des fibres et/ou aux paramètres d'élasticité, sans avoir à faire tourner la sonde de mesure et donc on peut effectuer aisément des mesures in vivo.

Dans divers modes de réalisation du procédé selon l'invention, le procédé peut comporter une ou plusieurs des caractéristiques des revendications 1 à 8.

Par ailleurs, l'invention a également pour objet un dispositif de caractérisation d'un milieu mou anisotrope selon la revendication 9.

Dans divers modes de réalisation du dispositif de caractérisation selon l'invention, le dispositif de caractérisation peut comporter une ou plusieurs des caractéristiques des revendications 10 à 13.

Enfin, l'invention a encore pour objet un ensemble de sonde ultrasonore pour un dispositif tel que défini par la revendication 14

BREVE DESCRIPTION DES DESSINS

D'autres caractéristiques et avantages de l'invention apparaîtront au cours de la description suivante d'un de ses modes de réalisation, donné à titre d'exemple non limitatif, en regard des dessins joints.

Sur les dessins :

- la figure 1 est une vue en coupe illustrant la mise en oeuvre d'un procédé de caractérisation selon une forme de réalisation de l'invention,
- la figure 2 est une vue en plan d'un ensemble de sonde ultrasonore visible sur la figure 1, et
- la figure 3 est une vue schématique d'un dispositif de caractérisation auquel appartient l'ensemble de sonde ultrasonore de la figure 2.

DESCRIPTION PLUS DETAILLEE

Sur les différentes figures, les mêmes références désignent des éléments identiques ou similaires.

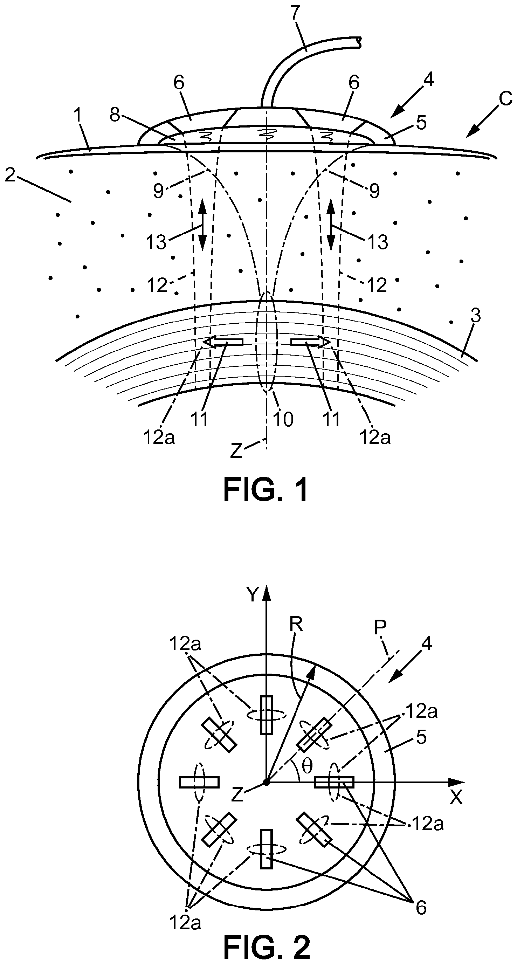

Comme représenté schématiquement sur les figures 1 et 2, l'invention a pour but de caractériser un milieu mou anisotrope tel qu'une partie d'un corps humain ou animal C, notamment vivant, comprenant au moins une partie 3 comportant des fibres et présentant une surface extérieure 1. Par exemple, la surface extérieure peut être la peau de l'être humain ou animal, et la partie 3 peut être une partie du coeur, notamment le myocarde, ou encore un muscle squelettique. La partie 3 peut éventuellement être séparée de la surface extérieure 1 par des tissus non fibreux 2.

Cette caractérisation est effectuée au moyen d'un ensemble de sonde 4 comportant une sonde d'excitation 5 et des sondes d'observations 6.

La sonde d'excitation 5 peut notamment être un transducteur ultrasonore d'excitation sensiblement en forme de disque ou de coupelle ayant un axe central Z disposé dans le sens de la profondeur du milieu mou anisotrope C lorsque la sonde d'excitation est en utilisation. Cette sonde d'excitation 5 comporte éventuellement une face concave destinée à être appliquée contre la surface extérieure 1, généralement avec remplissage de la concavité de cette face par un gel 8 classiquement utilisé en échographie. La sonde d'excitation 5 est adaptée pour émettre une onde ultrasonore de compression 9 dans le milieu mou anisotrope C selon ledit axe central Z, dont la tache focale est dans une zone centrale 10 s'étendant sur quelques centimètres le long de l'axe central Z (par exemple 1 à 6 cm) et ayant une largeur de quelques millimètres perpendiculairement à l'axe central Z (par exemple à 0,2 à 3 mm).

La position et la longueur de la tache focale 10 le long de l'axe central Z sont conçues pour que statistiquement, lorsque la sonde d'excitation est disposée contre la surface extérieure 1, la tache focale se trouve normalement au moins partiellement dans la partie fibreuse 3 à caractériser pour les êtres humains ou animaux examinés. Ainsi, pour la caractérisation du myocarde chez l'être humain, la tache focale 10 débute par exemple à une profondeur z de 2 à 4 cm et se termine par exemple à une profondeur z de 5 à 10 cm.

La sonde d'excitation 5 peut présenter par exemple un rayon R inférieur à 3 cm, avantageusement inférieur à 2cm.

Les sondes d'observation 6 peuvent être en nombre supérieur ou égal à 2, avantageusement supérieur ou égal à 3, par exemple compris entre 3 et 20, avantageusement entre 5 et 10. Ces sondes d'observation sont au nombre de 8 dans l'exemple représenté.

Les sondes d'observation 6 sont réparties autour de l'axe central Z, respectivement en correspondance avec des directions de propagation différentes P divergeant par rapport audit axe central Z. Dans tous les cas, les directions de propagation P comprennent au moins deux directions de propagation qui forment entre elles un angle différent de 0 degré et différent de 180 degrés.

Les sondes d'observation (6) peuvent être disposées à une distance inférieure à 2 cm dudit axe central Z, par exemple inférieure à 0,5 cm. Les sondes d'observation 6 peuvent être avantageusement équiréparties sur un cercle centré sur l'axe central Z. Dans ce qui suit, on repérera les directions de propagation divergentes P dans un repère orthonormé X, Y dans un plan perpendiculaire à l'axe central Z, par leur angle $\theta$ par rapport à l'axe X.

Les sondes d'observation 6 peuvent être chacune un transducteur ultrasonore d'observation ou éventuellement un groupe de transducteurs ultrasonores d'observation, du type classiquement utilisé en imagerie médicale, focalisés à relativement grande distance parallèlement à l'axe central Z de façon à émettre chacun une onde ultrasonore de compression formant un faisceau 12 parallèle à l'axe Z.

Avantageusement, chaque sonde d'observation 6 est formée par un unique transducteur ultrasonore d'observation. Les transducteurs ultrasonores d'observation 6 peuvent être conformés comme des barrettes s'étendant radialement par rapport à l'axe Z et présentant une certaine concavité vers le milieu C, de façon que la tache focale 12a de chaque transducteur ultrasonore d'observation 6 soit située environ à même profondeur que la tache focale 10 dans le milieu C, et de façon que cette tache focale 12a présente une faible dimension dans la direction radiale par rapport à l'axe Z, par exemple de l'ordre de 0,1 à 2 mm et une plus grande dimension, de l'ordre de quelques millimètres, en direction orthoradiale. Les taches focales 12a correspondant aux différents transducteurs ultrasonores d'observation sont de préférence séparés les uns des autres.

Avantageusement, les sondes d'observation 6 sont portées par la sonde d'excitation 5, et peuvent être par exemple incluses dans des évidements ménagés dans l'épaisseur de la sonde d'excitation 5.

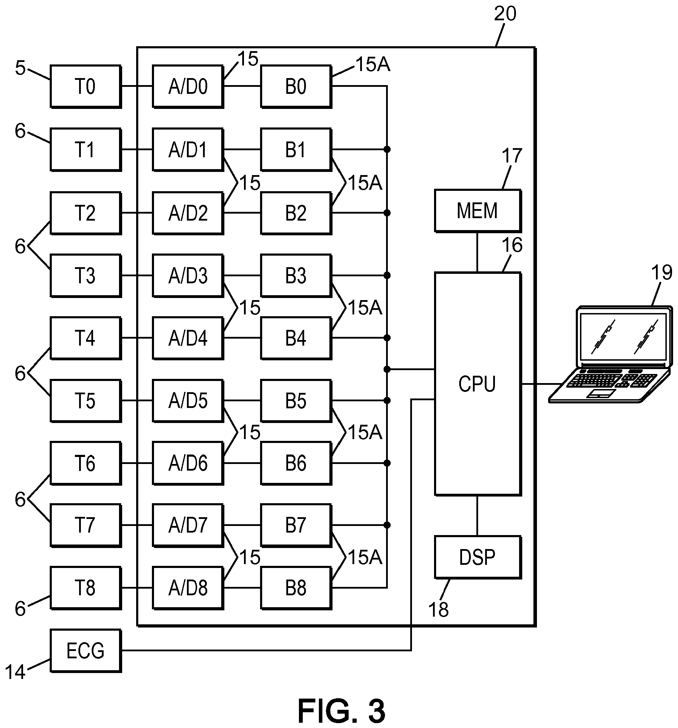

Les sondes d'excitation 5 et les sondes d'observation 6 communiquent avec un dispositif de commande, par exemple par l'intermédiaire d'un câble 7 multifilaire. Comme représenté sur la figure 3, ce dispositif de commande peut comprendre un ordinateur 19 ou similaire communiquant avec un boîtier électronique 20 spécifique, lui-même relié à l'ensemble de sonde 4 par le câble 7. On notera que le dispositif de commande pourrait éventuellement être un seul appareil intégrant toutes les fonctionnalités du boîtier électronique 20 et de l'ordinateur 19.

Dans l'exemple représenté, le boîtier électronique 20 peut comporter autant de voies que de transducteurs, par exemple 9 voies, reliées respectivement à la sonde d'excitation 5 (T0) et aux sondes d'observation 6 (T1-T8). Chacune de ces voies peut comporter un convertisseur analogique-digital 15 (A/D0-AD8) associé à une mémoire tampon 15A (B0-B8) et communiquant avec une unité centrale électronique 16 (CPU) telle qu'un microprocesseur ou similaire, qui elle-même peut communiquer par exemple avec une mémoire 17 (MEM) et un circuit de traitement de signal 18 (DSP), ainsi qu'avec l'ordinateur 19. L'unité centrale électronique 16 peut éventuellement communiquer en outre avec un appareil d'électrocardiogramme 14 (ECG).

Le dispositif qui vient d'être décrit fonctionne comme suit.

### (a) Etape de mesure

Lorsqu'un utilisateur veut caractériser la partie fibreuse 3 du milieu C, il applique l'ensemble de sonde 4 sur la peau 1 comme expliqué précédemment et lance une étape de mesure (a) au cours de laquelle l'unité centrale électronique 16 fait d'abord émettre une onde ultrasonore focalisée par la sonde d'excitation 5 pendant une courte durée, pour générer un déplacement des tissus le long de l'axe Z par effet de pression d'onde, par exemple sur le principe général expliqué dans le document WO2004/021038.

Cette onde de cisaillement 11 se propage sensiblement radialement par rapport à l'axe Z (voir la figure 1) et pendant cette propagation, l'unité centrale électronique 16 fait observer le milieu C simultanément par toutes les sondes d'observations 6, pour observer la propagation de l'onde de cisaillement 11. A cet effet, l'unité centrale électronique 16 fait émettre par toutes les sondes d'observation 6 simultanément, à cadence rapide (par exemple 300 tirs par seconde ou plus), des ondes ultrasonores de compression et lesdites sonde d'observation 6 captent les signaux ultrasonores réverbérés par les tissus 3, comme expliqué notamment dans les documents WO00/55616 et WO2004/021038, et ces signaux son mémorisés d'abord dans les mémoires tampons 15a puis dans la mémoire 17.

Cette étape de mesure dure par exemple quelques millisecondes.

Eventuellement, le boîtier électronique 20 pourrait comporter moins de voies que de transducteurs. Par exemple, le boîtier électronique 20 pourrait comporter une voie A/D0 - B0 pour le transducteur ultrasonore d'excitation 5 et une voie A/D1 - B1 reliée successivement aux différents transducteurs ultrasonores d'observation 6 par un dispositif de commutation (non représenté). Dans ce cas, l'étape de mesure (a) comporte plusieurs émissions successives d'onde de cisaillement suivies chacune par l'observation de sa propagation par l'une des sondes d'observation 6, le processus étant répété pour chaque sonde d'observation 6. Même dans ce cas, l'étape de mesure est très brève, inférieure à 50 ms.

### (b) Etape de calcul

Dans une étape ultérieure de calcul (b), effectuée en temps réel ou différé, on fait exploiter les signaux réverbérés mémorisés, par l'unité centrale électronique 16 ou l'ordinateur 19. Au cours de ce traitement, on utilise lesdits signaux pour déterminer des déplacement ou déformations internes des tissus 3 au cours du temps en regard de chaque sonde d'observation 6, par exemple par des calculs de corrélation sur les signaux captés, comme expliqué notamment dans les documents WO00/55616 et WO2004/021038.

On peut donc ainsi repérer le passage de l'onde élastique de cisaillement en regard de chaque sonde d'observation 6, et ce à chaque profondeur dans les tissus 3. Par exemple, le passage de l'onde de cisaillement peut ainsi être repéré à un nombre prédéterminé de profondeurs dans les tissus 3, par exemple entre 10 et 30 profondeurs, par exemple de millimètre en millimètre.

L'unité centrale électronique 16 ou l'ordinateur 19 détermine alors, à chaque profondeur, le temps de vol t de l'onde de cisaillement entre la sonde d'excitation 5 et chaque sonde d'observation 6, ou un autre paramètre représentatif du temps de vol, ou encore une autre donnée de propagation représentative de la propagation de l'onde de cisaillement, et en déduit la vitesse de propagation V de l'onde de cisaillement à la profondeur considérée dans la direction radiale P correspondant à chaque sonde d'observation 6.

### (c) Etape de caractérisation :

On peut ensuite déterminer au moins une caractéristique rhéologique du milieu 3, choisie parmi une direction des fibres, un paramètre rhéologique d'élasticité (notamment module d'élasticité) dans une direction perpendiculaire aux fibres et un paramètre rhéologique d'élasticité (notamment module d'élasticité) dans la direction des fibres. Avantageusement, ces caractéristiques rhéologiques sont déterminées en même temps.

Lesdites caractéristiques rhéologiques peuvent être déterminées par diverses méthodes, par exemple par interpolation ou plus préférentiellement par décomposition en valeurs singulières.

1. Interpolation

Dans cette méthode, on détermine par interpolation, en fonction des valeurs de la vitesse de propagation calculées à l'étape de calcul (b) dans chaque directions de propagation P, une courbe sensiblement ellipsoïdale $C(V(\theta).\cos\theta, V(\theta).\sin\theta)$ où $V(\theta)$ est la valeur du paramètre de propagation dans le plan X, Y. La direction des fibres correspond à un angle $\theta_0$ correspondant au maximum de $V(\theta)$, le paramètre rhéologique d'élasticité dans la direction des fibres est alors déterminé en fonction de $V(\theta_0)$ et le paramètre rhéologique d'élasticité dans la direction perpendiculaire aux fibres étant déterminé en fonction de $V(\theta_0+\pi/2)$. Par exemple, lorsque ces paramètres sont des modules d'élasticité Epar parallèlement aux fibres et Eperp perpendiculairement aux fibres, ces modules d'élasticité peuvent être déterminés par les formules la formule

$$V(\theta 0) = \sqrt{\frac{Epar}{3\rho}} \quad \text{et} \quad V(\theta 0 + \pi/2) = \sqrt{\frac{Eperp}{3\rho}}$$

, où $\rho$ est la densité du milieu 3.

2. Décomposition en valeurs singulières

Dans cette méthode, on détermine d'abord à chaque profondeur, à partir des vitesses de propagation mesurées, le tenseur élastique de propagation de l'onde ultrasonore dans le milieu 3.

Ce tenseur est une matrice M ici de rang 2 puisque la propagation se fait uniquement dans le plan X, Y :

$$M = \begin{bmatrix} E_{XX}, E_{XY} \\ E_{XY}, E_{YY} \end{bmatrix}$$

, où les composantes E sont des modules d'élasticité. Ce tenseur élastique est défini notamment par Royer et Dieulesaint (2000 ; Elastic Waves in Solids I: Free and Guided Propagation: Springer-Verlag Berlin Heidelberg) .

Comme expliqué dans l'article *Lee et al.* susmentionné (W.-N. Lee, B. Larrat, M. Pernot, and M. Tanter, "Ultrasound Elastic Tensor Imaging: Comparison with MR Diffusion Tensor Imaging in the Myocardium," Physics in Medicine and Biology, vol. 57, pp. 5075-5095, 2012), le tenseur M peut être déterminé à partir des vitesses $V(\theta)$ déterminées à l'étape de calcul (b), en résolvant l'équation :

$$\begin{bmatrix} \rho V(\theta_1)^2 \\ \vdots \\ \rho V(\theta_N)^2 \end{bmatrix} = \begin{bmatrix} \cos^2\theta_1 & 2\cos\theta_1\sin\theta_1 & \sin^2\theta_1 \\ & \vdots & \\ \cos^2\theta_N & 2\cos\theta_N\sin\theta_N & \sin^2\theta_N \end{bmatrix} \begin{bmatrix} E_{XX} \\ E_{XY} \\ \mu_{YY} \end{bmatrix} \qquad (1)$$

où $\rho$ est la densité du milieu 3, N le nombre de transducteurs et $\theta 1 .. \theta N$ sont les angles des différentes directions de propagation P correspondant aux sondes d'observation 6.

Toujours comme expliqué l'article *Lee et al.* susmentionné, le tenseur M peut être ensuite décomposé en valeurs singulières pour obtenir une matrice diagonale M0 donnant les paramètres Epar, Eperp susmentionnés :

$$M0 = \begin{bmatrix} E_{PAR} & 0 \\ 0 & E_{PERP} \end{bmatrix}.$$

Cette détermination s'accompagne de la détermination de la direction $\theta 0$ des fibres à la profondeur considérée, puisqu'on a la relation :

$M = R.M0.R^T$ , où R est la matrice de rotation correspondant à l'angle $\theta 0$ :

$$R = \begin{bmatrix} \cos\theta_0 & -\sin\theta_0 \\ \sin\theta_0 & \cos\theta_0 \end{bmatrix}.$$

Les étapes de mesure (a), de calcul (b) et de caractérisation (c), peuvent être réitérées à cadence relativement rapide, de façon à suivre le coeur ou autre muscle dans son fonctionnement et à donner des valeurs successives des caractéristiques rhéologiques paramètres du milieu 3 par exemple sur un cycle complet de fonctionnement du muscle formant le milieu 3. Lorsqu'il s'agit du coeur, les mesures successives peuvent être synchronisées avec le cycle cardiaque grâce aux données communiquées par l'électrocardiographe 14 à l'unité centrale électronique 16. On en déduit une valeur d'un paramètre physiologique lié à la contraction du muscle, par exemple contractilité, dureté maximale ou autre.

On notera que les signaux captés par les sondes d'observation peuvent également permettre de recaler les mesures successives les unes par rapport aux autres en repérant les déformations ou déplacements du milieu 3 dues au fonctionnement du muscle formant ce milieu 3.

**EP 3 099 240 B1**

**Revendications**

1. Procédé de caractérisation d'un milieu mou anisotrope (C) comprenant au moins une partie (3) comportant des fibres et présentant une surface extérieure (1), ce procédé comprenant les étapes suivantes :

   (a) une étape de mesure au cours de laquelle on génère au moins une onde de cisaillement (11) qui se propage en divergeant à partir d'une zone centrale (10) dans le milieu mou anisotrope et, on observe avec des transducteurs ultrasonores d'observation (6), à partir de la surface (1) du milieu mou anisotrope, une propagation de ladite au moins une onde de cisaillement dans plusieurs directions de propagation prédéterminées (P) à partir de ladite zone centrale (10), en maintenant fixes les transducteurs ultrasonores d'observation (6), lesdites directions de propagation (P) prédéterminées comprenant au moins deux directions faisant entre elles un angle différent de 0 degré et différent de 180 degrés, lesdits transducteurs ultrasonores d'observation (6) étant disposés au moins selon lesdites directions de propagation (P) prédéterminées et ladite étape de mesure étant effectuée en une durée inférieure à 50 ms ;
   (b) au moins une étape de calcul au cours de laquelle on détermine, à partir de données collectées au cours de l'étape de mesure (a), au moins un paramètre de propagation de l'onde de cisaillement dans chacune desdites directions de propagation (P) prédéterminées ;
   (c) une étape de caractérisation au cours de laquelle, à partir dudit au moins un paramètre de propagation de l'onde de cisaillement déterminé dans chacune des directions de propagation (P) à l'étape de calcul (b), on détermine au moins une caractéristique rhéologique du milieu mou anisotrope, choisie parmi une direction des fibres du milieu mou anisotrope, un paramètre rhéologique d'élasticité dans une direction perpendiculaire aux fibres et un paramètre rhéologique d'élasticité dans la direction des fibres.

2. Procédé selon la revendication 1, dans lequel les paramètres rhéologiques d'élasticité déterminés au cours de l'étape de caractérisation (c) sont des modules d'élasticité.

3. Procédé selon l'une quelconque des revendications précédentes, dans lequel :

   - au cours de l'étape de mesure (a), on génère l'onde de cisaillement sur une certaine plage de profondeurs dans le milieu mou anisotrope et on observe la propagation de ladite au moins une onde de cisaillement à différentes profondeurs à l'intérieur de ladite plage de profondeurs,
   - au cours de l'étape de calcul (b), on détermine ledit au moins un paramètre de propagation de l'onde de cisaillement dans chacune desdites directions de propagation (P) prédéterminées auxdites différentes profondeurs,
   - et au cours de l'étape de caractérisation (c), on détermine ladite au moins une caractéristique rhéologique auxdites différentes profondeurs.

4. Procédé selon l'une quelconque des revendications précédentes, dans lequel au cours de l'étape de mesure (a), les directions de propagation prédéterminées (P) dans lesquelles on observe la propagation de l'onde de cisaillement, sont en nombre compris entre 3 et 20.

5. Procédé selon l'une quelconque des revendications précédentes, dans lequel, au cours de l'étape de mesure (a), on observe la propagation de ladite au moins une onde de cisaillement simultanément dans toutes lesdites directions de propagation prédéterminées (P), avec tous les transducteurs ultrasonores d'observation (6) en même temps.

6. Procédé selon l'une quelconque des revendications 1 à 4, dans lequel, au cours de l'étape de mesure (a), on émet successivement plusieurs ondes de cisaillement et on observe successivement la propagation de chaque onde de cisaillement dans au moins une desdites directions de propagation prédéterminées (P), avec une partie des transducteurs ultrasonores d'observation (6).

7. Procédé selon l'une quelconque des revendications précédentes, dans lequel :

   - au cours de l'étape de mesure (a), on détecte le passage de l'onde de cisaillement en des points de mesure prédéterminés disposés respectivement selon lesdites directions de propagation prédéterminées (P),
   - et au cours de l'étape de calcul (b), le paramètre de propagation de l'onde de cisaillement déterminé est un paramètre représentatif soit d'une vitesse de propagation de l'onde de cisaillement, soit d'un temps de vol de l'onde de cisaillement depuis la zone centrale (10).

7

8. Procédé selon la revendication 7, dans lequel l'onde de cisaillement est générée à partir d'un axe central (Z) perpendiculaire à la surface (1) du milieu mou anisotrope et lesdits points de mesure (6) où on détecte le passage de l'onde de cisaillement, sont situés chacun à une distance dudit axe central inférieure à 2 cm.

9. Dispositif de caractérisation d'un milieu mou anisotrope ayant au moins une partie comprenant des fibres et présentant une surface extérieure, ce dispositif de caractérisation comprenant un dispositif de commande électronique (19, 20) qui commande une sonde d'excitation (5) et des transducteurs ultrasonores d'observation (6), la sonde d'excitation (5) étant adaptée pour générer une onde de cisaillement dans le milieu mou anisotrope à partir d'une zone centrale (10) et les transducteurs ultrasonores d'observation (6) étant disposés selon plusieurs directions de propagation (P) prédéterminées à partir de ladite zone centrale (10), lesdites directions de propagation (P) prédéterminées comprenant au moins deux directions faisant entre elles un angle différent de 0 degré et différent de 180 degrés, le dispositif de commande électronique (19, 20) étant adapté pour, lorsque la sonde d'excitation (5) et les transducteurs ultrasonores d'observation (6) sont disposés à la surface du milieu mou anisotrope :

   (a) faire générer par la sonde d'excitation (5), au moins une onde de cisaillement adaptée pour se propager en divergeant à partir de la zone centrale (10) dans le milieu mou anisotrope, en maintenant fixes les transducteurs ultrasonores d'observation (6) et faire observer par les transducteurs ultrasonores d'observation (6), en une durée totale d'observation inférieure à 50 ms, une propagation de l'onde de cisaillement dans lesdites directions de propagation (P) prédéterminées à partir de ladite zone centrale (10) ;
   (b) déterminer, à partir de données collectées par les transducteurs ultrasonores d'observation (6), au moins un paramètre de propagation de l'onde de cisaillement simultanément dans chacune desdites directions de propagation (P) prédéterminées ;
   (c) déterminer, à partir dudit au moins un paramètre de propagation de l'onde de cisaillement dans chacune des directions de propagation (P) prédéterminées, au moins une caractéristique rhéologique du milieu mou anisotrope, choisie parmi une direction des fibres du milieu mou anisotrope, un paramètre rhéologique d'élasticité dans une direction perpendiculaire aux fibres et un paramètre rhéologique d'élasticité dans la direction des fibres.

10. Dispositif selon la revendication 9, dans lequel lesdits transducteurs ultrasonores d'observation sont en nombre compris entre 3 et 20.

11. Dispositif selon la revendication 10, dans lequel le dispositif de commande électronique (19, 20) est adapté pour détecter le passage de l'onde de cisaillement en regard de chaque transducteur ultrasonore d'observation (6) et le paramètre de propagation de l'onde de cisaillement, déterminé par l'unité centrale, est un paramètre représentatif soit d'une vitesse de propagation de l'onde de cisaillement, soit d'un temps de vol de l'onde de cisaillement.

12. Dispositif selon la revendication 11, dans lequel le dispositif de commande électronique (19, 20) est adapté pour détecter le passage de l'onde de cisaillement :

   en faisant émettre par les transducteurs ultrasonores d'observation (6), des ondes acoustiques de compression à une cadence d'au moins 300 tirs d'ondes ultrasonores de compression par seconde,
   en faisant capter des signaux ultrasonores réverbérés par le milieu mou anisotrope par les transducteurs ultrasonores d'observation (6),
   et en déterminant ainsi des déplacements internes dudit milieu mou anisotrope au passage de l'onde de cisaillement en regard desdits transducteurs ultrasonores d'observation (6).

13. Dispositif selon la revendication 12, dans lequel la sonde d'excitation (5) est un transducteur ultrasonore sensiblement en forme de disque qui porte les transducteurs ultrasonores d'observation (6).

14. Ensemble de sonde ultrasonore pour un dispositif de caractérisation selon l'une quelconque des revendications 9 à 13, comportant un transducteur ultrasonore d'excitation (5) sensiblement en forme de disque et adapté pour émettre une onde ultrasonore de compression selon un axe central (Z) afin de déplacer le milieu mou anisotrope selon ledit axe central par pression de radiation et faire ainsi propager dans le milieu une onde de cisaillement divergeant à partir dudit axe central, ledit transducteur ultrasonore d'excitation portant des transducteurs ultrasonores d'observation (6) répartis respectivement dans des directions de propagation (P) différentes divergeant par rapport audit axe central (Z) et disposés à une distance inférieure à 2 cm dudit axe central, chaque transducteur ultrasonore d'observation étant adapté pour émettre des ondes de compression en forme de faisceau (12) parallèle à l'axe central (Z), lesdites directions de propagation (P) comprenant au moins deux directions faisant entre elles un angle différent de 0 degré et différent de 180 degrés.

**15.** Ensemble de sonde ultrasonore selon la revendication 14, dans lequel les transducteurs ultrasonores d'observation (6) sont équirépartis sur un cercle centré sur l'axe central (Z).

**Patentansprüche**

**1.** Verfahren zur Charakterisierung eines anisotropen weichen Mediums (C), das mindestens einen Teil (3) mit Fasern umfasst und eine Außenfläche (1) aufweist, wobei das Verfahren die folgenden Schritte umfasst:

(a) einen Messschritt, bei dem mindestens eine Scherwelle (11) erzeugt wird, die sich von einem zentralen Bereich (10) in dem anisotropen weichen Medium aus divergierend ausbreitet, und mit Ultraschallwandlern zur Beobachtung (6), ausgehend von der Oberfläche (1) des anisotropen weichen Mediums eine Ausbreitung der mindestens einen Scherwelle in mehreren vorbestimmten Ausbreitungsrichtungen (P) von dem zentralen Bereich (10) aus beobachtet wird, wobei die Ultraschallwandler zur Beobachtung (6) feststehend gehalten werden, die vorbestimmten Ausbreitungsrichtungen (P) mindestens zwei Richtungen umfassen, die einen Winkel ungleich 0 Grad und ungleich 180 Grad zueinander bilden, wobei die Ultraschallwandler zur Beobachtung (6) zumindest entlang der vorbestimmten Ausbreitungsrichtungen (P) angeordnet sind, und wobei der Messschritt in einer Zeitdauer von weniger als 50 ms durchgeführt wird;
(b) mindestens einen Berechnungsschritt, bei dem aus den während des Messschritts (a) gesammelten Daten mindestens ein Ausbreitungsparameter der Scherwelle in jeder der vorbestimmten Ausbreitungsrichtungen (P) bestimmt wird;
(c) einen Charakterisierungsschritt, in dessen Verlauf ausgehend von dem mindestens einen Ausbreitungsparameter der Scherwelle, der in jeder der Ausbreitungsrichtungen (P) im Berechnungsschritt (b) bestimmt wurde, mindestens eine rheologische Kenngröße des anisotropen weichen Mediums, ausgewählt aus einer Faserrichtung des anisotropen weichen Mediums, ein rheologischer Parameter der Elastizität in einer Richtung senkrecht zu den Fasern und ein rheologischer Parameter der Elastizität in der Faserrichtung, bestimmt wird.

**2.** Verfahren nach Anspruch 1, wobei die im Charakterisierungsschritt (c) bestimmten rheologischen Elastizitätsparameter Elastizitätsmodule sind.

**3.** Verfahren nach einem der vorherigen Ansprüche, wobei:

- in dem Messschritt (a) die Scherwelle über einen bestimmten Tiefenbereich in dem anisotropen weichen Medium erzeugt wird und die Ausbreitung der mindestens einen Scherwelle in verschiedenen Tiefen innerhalb des Tiefenbereichs beobachtet wird,
- in dem Berechnungsschritt (b) der mindestens eine Ausbreitungsparameter der Scherwelle in jeder der vorbestimmten Ausbreitungsrichtungen (P) in den verschiedenen Tiefen bestimmt wird,
- und in dem Charakterisierungsschritt (c) die mindestens eine rheologische Eigenschaft bei den verschiedenen Tiefen bestimmt wird.

**4.** Verfahren nach einem der vorhergehenden Ansprüche, wobei während des Messschritts (a) die vorbestimmten Ausbreitungsrichtungen (P), in denen die Ausbreitung der Scherwelle beobachtet wird, in einer Anzahl zwischen 3 und 20 vorliegen.

**5.** Verfahren nach einem der vorhergehenden Ansprüche, bei dem während des Messschritts (a) die Ausbreitung der mindestens einen Scherwelle gleichzeitig in allen vorbestimmten Ausbreitungsrichtungen (P) mit allen Ultraschallwandlern zur Beobachtung (6) gleichzeitig beobachtet wird.

**6.** Verfahren nach einem der Ansprüche 1 bis 4, bei dem während des Messschritts (a) nacheinander mehrere Scherwellen erzeugt werden und die Ausbreitung jeder Scherwelle in mindestens einer der vorbestimmten Ausbreitungsrichtungen (P) nacheinander mit einem Teil der Ultraschallwandler zur Beobachtung (6) beobachtet wird.

**7.** Verfahren nach einem der vorherigen Ansprüche, wobei:

- während des Messschritts (a) der Durchgang der Scherwelle an vorbestimmten Messpunkten erfasst wird, die jeweils entlang der vorbestimmten Ausbreitungsrichtungen (P) angeordnet sind,
- und in dem Berechnungsschritt (b) der bestimmte Scherwellenausbreitungsparameter ein Parameter ist, der entweder für eine Ausbreitungsgeschwindigkeit der Scherwelle oder eine Flugzeit der Scherwelle vom zentralen Bereich (10) repräsentativ ist.

8. Verfahren nach Anspruch 7, wobei die Scherwelle von einer Mittelachse (Z) aus erzeugt wird, die senkrecht zur der Oberfläche (1) des anisotropen weichen Mediums verläuft und die Messpunkte (6), an denen der Durchgang der Scherwelle erfasst wird, jeweils in einem Abstand von der Mittelachse von weniger als 2 cm angeordnet sind.

9. Vorrichtung zur Charakterisierung eines anisotropen weichen Mediums, das zumindest in einem Teil Fasern enthält und eine äußere Oberfläche aufweist, wobei die Vorrichtung zur Charakterisierung eine elektronische Steuereinrichtung (19, 20) umfasst, die eine Erregersonde (5) und Ultraschallwandler zur Beobachtung (6) steuert, wobei die Erregersonde (5) so ausgelegt ist, dass sie eine Scherwelle in dem anisotropen weichen Medium ausgehend von einem zentralen Bereich (10) erzeugt, und die Ultraschallwandler zur Beobachtung (6) in mehreren Ausbreitungsrichtungen (P) ausgehend vom zentralen Bereich (10) angeordnet sind, wobei die vorbestimmten Ausbreitungsrichtungen (P) mindestens zwei Richtungen umfassen, die untereinander einen Winkel bilden, der von 0 Grad und von 180 Grad verschieden ist, wobei die elektronische Steuereinrichtung (19, 20) so ausgelegt ist, dass sie, wenn die Erregersonde (5) und die Ultraschallwandler zur Beobachtung (6) an der Oberfläche des anisotropen weichen Mediums angeordnet sind, folgendes tut:

(a) mindestens eine Scherwelle erzeugen durch die Erregersonde (5), die sich divergierend von dem zentralen Bereich (10) in dem anisotropen weichen Medium ausbreiten kann, während die Ultraschallwandler zur Beobachtung (6) stationär gehalten werden und durch die Ultraschallwandler zur Beobachtung (6) in einer Gesamtbeobachtungszeit von weniger als 50 ms eine Ausbreitung der Scherwelle in den vorbestimmten Ausbreitungsrichtungen (P) von dem zentralen Bereich (10) aus beobachten zu lassen;
(b) aus den von den Ultraschallwandlern zur Beobachtung (6) gesammelten Daten mindestens einen Ausbreitungsparameter der Scherwelle gleichzeitig in jeder der vorbestimmten Ausbreitungsrichtungen (P) zu bestimmen;
(c) bestimmen, ausgehend von dem mindestens einen Ausbreitungsparameter der Scherwelle in jeder der vorbestimmten Ausbreitungsrichtungen (P), mindestens einer rheologischen Eigenschaft des anisotropen weichen Mediums, ausgewählt aus einer Faserrichtung des anisotropen weichen Mediums, einem rheologischen Parameter der Elastizität in einer Richtung senkrecht zu den Fasern und einem rheologischen Parameter der Elastizität in der Faserrichtung.

10. Vorrichtung nach Anspruch 9, wobei die Anzahl der Ultraschallwandler zur Beobachtung zwischen 3 und 20 liegt.

11. Vorrichtung nach Anspruch 10, bei der die elektronische Steuereinrichtung (19, 20) so ausgelegt ist, dass sie den Durchgang der Scherwelle gegenüber jedem Ultraschallwandler zur Beobachtung (6) erfasst und der Ausbreitungsparameter der Scherwelle, der von der Zentraleinheit bestimmt wird, ein Parameter ist, der entweder für eine Ausbreitungsgeschwindigkeit der Scherwelle oder für eine Flugzeit der Scherwelle repräsentativ ist.

12. Vorrichtung nach Anspruch 11, bei der die elektronische Steuereinrichtung (19, 20) so beschaffen ist, dass sie den Durchgang der Scherwelle erfasst:

indem die Ultraschallwandler zur Beobachtung (6) veranlasst werden, akustische Kompressionswellen mit einer Rate von mindestens 300 Schüssen von Kompressionsultraschallwellen pro Sekunde auszusenden,
indem man die Ultraschallwandler zur Beobachtung (6) veranlasst, Ultraschallsignale zu empfangen, die von dem anisotropen weichen Medium zurückgeworfen werden,
und so innere Verschiebungen des anisotropen weichen Mediums beim Durchgang der Scherwelle gegenüber den Ultraschallwandlern zur Beobachtung (6) bestimmt werden.

13. Vorrichtung nach Anspruch 12, wobei die Erregersonde (5) ein im Wesentlichen scheibenförmiger Ultraschallwandler ist, der die Ultraschallwandler zur Beobachtung (6) trägt.

14. Ultraschallsondenanordnung für eine Charakterisierungsvorrichtung nach einem der Ansprüche 9 bis 13, mit einem im Wesentlichen scheibenförmigen Erreger-Ultraschallwandler (5), der so beschaffen ist, dass er eine Kompressionsultraschallwelle entlang einer Mittelachse (Z) aussendet, um das anisotrope weiche Medium entlang der Mittelachse durch Strahlungsdruck zu platzieren und so eine von der Mittelachse divergierende Scherwelle in dem Medium zu verbreiten, wobei der Erreger-Ultraschallwandler Ultraschallwandler zur Beobachtung (6) trägt, die jeweils in verschiedenen Ausbreitungsrichtungen (P) verteilt sind, die von der Mittelachse (Z) divergieren, und in einem Abstand von weniger als 2 cm von der Mittelachse angeordnet sind, wobei jeder Beobachtungs-Ultraschallwandler dazu ausgelegt ist Kompressionswellen in Form eines Strahls (12) parallel zur Mittelachse (Z) auszusenden, wobei die Ausbreitungsrichtungen (P) mindestens zwei Richtungen umfassen, die zwischen sich einen von 0 und von 180 Grad verschiedenen Winkel einschließen.

15. Ultraschallsondenanordnung nach Anspruch 14, bei der die Ultraschallwandler zur Beobachtung (6) auf einem Kreis, der auf der Mittelachse (Z) zentriert ist, gleichmäßig verteilt sind.

**Claims**

1. A method for characterizing an anisotropic soft medium (C) comprising at least one portion (3) including fibers and having an outer surface (1), this method comprising the following steps:

   (a) a measurement step during which at least one shear wave (11) is generated which propagates by diverging from a central area (10) in the anisotropic soft medium and, a propagation of said at least one shear wave is observed with ultrasonic observation transducers (6), from the surface (1) of the anisotropic soft medium, in several predetermined propagation directions (P) from said central area (10) by maintaining fixed the ultrasonic observation transducers (6), said predetermined propagation directions (P) comprising at least two directions forming between them an angle different from 0 degree and different from 180 degrees, said ultrasonic observation transducers (6) being positioned at least along said predetermined propagation directions (P) and said measurement step being carried out within a period of less than 50 ms;
   (b) at least one computing step during which at least one propagation parameter of the shear wave is determined, from data collected during the measurement step (a) in each of said predetermined propagation directions (P);
   (c) a characterization step during which, from said at least one propagation parameter of the shear wave, determined in each of the propagation directions (P) in the computing step (b), at least one rheological characteristic of the anisotropic soft medium is determined, selected from among a direction of the fibers of the anisotropic soft medium, a rheological elasticity parameter in a direction perpendicular to the fibers and a rheological elasticity parameter in the direction of the fibers.

2. The method according to claim 1, wherein the rheological elasticity parameters determined during the characterization step (c) are elasticity moduli.

3. The method according to any one of the previous claims, wherein:

   - during the measurement step (a), the shear wave is generated over a certain range of depths in the anisotropic soft medium and the propagation of said at least one shear wave is observed at different depths inside said range of depths,
   - during the computing step (b), said at least one propagation parameter of the shear wave is determined in each of said predetermined propagation directions (P) at said different depths,
   - and during the characterization step (c), said at least one rheological characteristic is determined at said different depths.

4. The method according to any one of the previous claims, wherein during the measurement step (a), the predetermined propagation directions (P) in which the propagation of the shear wave is observed, are in a number comprised between 3 and 20.

5. The method according to any one of the previous claims, wherein, during the measurement step (a), the propagation of said at least one shear wave is simultaneously observed in all of said predetermined propagation directions (P), with all the ultrasonic observation transducers (6) at the same time.

6. The method according to any one of claims 1 to 4, wherein, during the measurement step (a), several shear waves are successively emitted and the propagation of each shear wave is successively observed in at least one of said predetermined propagation directions (P), with a portion of the ultrasonic observation transducers (6).

7. The method according to any one of the previous claims, wherein:

   - during the measurement step (a), the passing of the shear wave in predetermined measurement points respectively positioned along said predetermined propagation directions (P) is detected,

- and during the computing step (b), the determined propagation parameter of the shear wave is a representative parameter of either a propagation velocity of the shear wave, or a time of flight of the shear wave from the central area (10).

8. The method according to claim 7, wherein the shear wave is generated from a central axis (Z) perpendicular to the surface (1) of the anisotropic soft medium and said measurement points (6) where the passing of the shear wave is detected, are each located at a distance from said central axis of less than 2 cm.

9. A device for characterizing an anisotropic soft medium having at least one portion comprising fibers and having an outer surface, the characterization device comprising an electronic control device (19, 20) which controls an excitation probe (5) and ultrasonic observation transducers (6), the excitation probe (5) being adapted for generating a shear wave in the anisotropic soft medium from a central area (10) and the ultrasonic observation transducers (6) being positioned along several predetermined propagation directions (P) from said central area (10), said predetermined propagation directions (P) comprising at least two directions forming between them an angle different from 0 degrees and different from 180 degrees, the electronic control device (19, 20) being adapted for, when the excitation probe (5) and the ultrasonic observation transducers (6) are positioned at the surface of the anisotropic soft medium :

    (a) have the excitation probe (5), generate at least one shear wave adapted for propagating while diverging from the central area (10) into the anisotropic soft medium and having the ultrasonic observation transducers (6) observe, within a total period of observation of less than 50 ms, a propagation of the shear wave in said predetermined propagation direction (P) from said central area (10);
    (b) determining, from data collected by the ultrasonic observation transducers (6), at least one propagation parameter of the shear wave simultaneously in each of said predetermined propagation directions (P);
    (c) determining, from said at least one propagation parameter of the shear wave in each of the predetermined propagation directions (P), at least one rheological characteristic of the anisotropic soft medium, selected from among a direction of the fibers of the anisotropic soft medium, a rheological elasticity parameter in a direction perpendicular to the fibers and a rheological elasticity parameter in the direction of the fibers.

10. The device according to claim 9, wherein said ultrasonic observation transducers are in a number comprised between 3 and 20.

11. The device according to claim 10, wherein the electronic control device (19, 20) is adapted for detecting the passing of the shear wave facing each ultrasonic observation transducer (6) and the propagation parameter of the shear wave, determined by the central unit, is a representative parameter of either a propagation velocity of the shear wave, or of a time of flight of the shear wave.

12. The device according to claim 11, wherein the electronic control device (19, 20) is adapted for detecting the passing of the shear wave:

    by having the ultrasonic observation transducers (6) emit acoustic compressional waves at a rate of at least 300 shots of ultrasonic compressional waves per second,
    by having ultrasonic signals reflected by the anisotropic soft medium sensed by the ultrasonic observation transducers (6),
    and by thereby determining internal displacements of said anisotropic soft medium upon passing of the shear wave facing said ultrasonic observation transducers (6).

13. The device according to claim 12, wherein the excitation probe (5) is an ultrasonic substantially disk-shaped transducer which bears the ultrasonic observation transducers (6).

14. An ultrasonic probe set for a characterization device according to any of claims 9 to 13, including an ultrasonic excitation transducer (5) substantially disk-shaped and adapted for emitting an ultrasonic compressional wave along a central axis (Z) in order to displace the anisotropic soft medium along said central axis by pressure of radiation and thus causing propagation in the medium of a divergent shear wave from said central axis, said ultrasonic excitation transducer bearing ultrasonic observation transducers (6) respectively distributed in different divergent propagation directions (P) with respect to said central axis (Z) and positioned at a distance of less than 2 cm from said central axis, each ultrasonic observation transducer being adapted for emitting compressional waves as a beam (12) parallel to the central axis (Z), said propagation directions (P) comprising at least two directions forming between them an angle different from 0 degree and different from 180 degrees.

15. The ultrasonic probe set according to claim 14, wherein the ultrasonic observation transducers (6) are equidistributed on a circle centered on the central axis (Z).

**FIG. 1**

**FIG. 2**

FIG. 3

## RÉFÉRENCES CITÉES DANS LA DESCRIPTION

**Documents brevets cités dans la description**

- WO 2004021038 A

- WO 0055616 A

**Littérature non-brevet citée dans la description**

- **W.-N. LEE ; M. PERNOT ; M. COUADE ; E. MESSAS ; P. BRUNEVAL ; A. BEL ; A. A. HAGÈGE ; M. FINK ; M. TANTER.** Mapping Myocardial Fiber Orientation Using Echocardiography-Based Shear Wave Imaging. *IEEE Trans. Med. Imaging,* 2012, vol. 31, 554-562

- **W.-N. LEE ; B. LARRAT ; M. PERNOT ; M. TANTER.** Ultrasound Elastic Tensor Imaging: Comparison with MR Diffusion Tensor Imaging in the Myocardium. *Physics in Medicine and Biology,* 2012, vol. 57, 5075-5095

- **ROYER ; DIEULESAINT.** Elastic Waves in Solids I: Free and Guided Propagation. Springer-Verlag, 2000